(19) Europäisches Patentamt
European Patent Office
Office européen des brevets

(11) **EP 3 842 081 A1**

(12) # EUROPEAN PATENT APPLICATION
published in accordance with Art. 153(4) EPC

(43) Date of publication:
**30.06.2021 Bulletin 2021/26**

(21) Application number: **19852239.3**

(22) Date of filing: **21.08.2019**

(51) Int Cl.:
**A61M 1/16** $^{(2006.01)}$

(86) International application number:
**PCT/JP2019/032665**

(87) International publication number:
**WO 2020/040208 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **23.08.2018 JP 2018156641**

(71) Applicant: **Nipro Corporation**
**Osaka-shi, Osaka 531-8510 (JP)**

(72) Inventors:
• **SHINZATO Toru**
**Toyohashi-shi, Aichi 441-8107 (JP)**
• **MIWA Masamiki**
**Kitanagoya-shi, Aichi 481-0042 (JP)**
• **MIZUNO Wataru**
**Osaka-shi, Osaka 531-8510 (JP)**

(74) Representative: **Söllner, Udo**
**Patentanwalt**
**Freundorferstrasse 34c**
**85598 Baldham (DE)**

(54) **APPARATUS FOR CALCULATING EXTRACELLULAR FLUID VOLUME AND METHOD FOR CALCULATING EXTRACELLULAR FLUID VOLUME**

(57) This apparatus for calculating extracellular fluid volume is provided with: an acquisition unit capable of acquiring a membrane surface area of a dialyzer used for dialysis; and a calculation unit for calculating an extracellular fluid volume after dialysis on the basis of the uric acid balance before and after the dialysis. The calculation unit calculates the removal volume of uric acid that is removed through the dialysis, on the basis of the overall uric acid mass transfer-area coefficient of the dialyzer, and calculates the overall uric acid mass transfer-area coefficient on the basis of the membrane surface area of the dialyzer that is acquired from the acquisition unit.

FIG. 5

```
          Start
            │
            ▼                    S12
  Acquire Various Types of Information
     About Hemodialysis Patient
            │
            ▼                    S14
  Acquire Various Types of Information
        About Hemodialysis
            │
            ▼                    S16
    Calculate Dialyzer Overall Mass
  Transfer-Area Coefficient for Uric Acid
            │
            ▼                    S18
  Calculate Clearance for Uric Acid of Dialyzer
            │
            ▼                    S20
    Calculate Removal Amount of Uric Acid
            │
            ▼                    S22
     Calculate Post-Hemodialysis
      Extracellular Fluid Volume
            │
            ▼
           End
```

EP 3 842 081 A1

## Description

Technical Field

The technique disclosed herein relates to calculation of extracellular fluid volume.

Background Art

Since kidneys malfunction in hemodialysis patients, all of the water they take in accumulates in their bodies. When the accumulated water in the bodies is removed by a hemodialysis procedure, the water is removed until a water volume in extracellular compartment (which may be referred to as an extracellular fluid volume, hereinbelow) becomes theoretically equal to an extracellular fluid volume of persons with normally functioning kidneys. In fact, however, a method for measuring an extracellular fluid volume has not been established. Thus, at present, a body weight of a patient whose extracellular fluid volume is estimated to be equal to that of a person with normally functioning kidneys is defined as a dry weight, and water in the body is removed until the patient's body weight becomes equal to the dry weight (Luik A, et al: Blood pressure control and fluid state in patients on long treatment time dialysis. J Am Soc Nephrol 5: 521, 1994). The dry weight is determined through a trial and error process based on clinical symptoms such as whether edema is observed or not, blood pressure level, whether a drop in blood pressure is observed during hemodialysis or not, whether the patient feels fatigued after hemodialysis or not, whether a muscle cramp is observed in a time window from a latter part of hemodialysis to post-hemodialysis or not, and the like (Jaeger JQ and Mehta RL: Assessment of Dry Weight in Hemodialysis: An Overview. J Am Soc Nephrol 10: 392-403, 1999). In fact, however, evaluation results based on such clinical symptoms may be incorrect (Charra B, et al: Clinical assessment of dry weight. Nephrol Dial Transplant 11 (Suppl 2): 16-19, 1996). Further, since body fat amount and/or muscle mass change(s) as some time elapses, the determined dry weight may not be used for a long time (Jaeger JQ and Mehta RL: Assessment of Dry Weight in Hemodialysis: An Overview. J Am Soc Nephrol 10: 392-403, 1999).

At present, one of methods for assessing whether a patient's post-hemodialysis weight is equal to his/her dry weight is to check if edema occurs or not. It is considered that edema occurs when an extracellular fluid volume of a patient is greater by 3 to 5 kg than that of a person with normally functioning kidneys (Gunal AI: How to determine 'dry weight'? Kidney Int 3: 377-379, 2013). This means that even if the extracellular fluid volume of patient at the set dry weight is greater than that of the person with normally functioning kidneys, it is considered that edema is not occurring if the difference is equal to or less than 3 to 5 kg. Thus, determining whether the dry weight is too high or not based on the presence/absence of edema may result in that the dry weight is set higher than it should be. When water is excessively accumulated in the body, volume of blood, which is a part of extracellular fluid, also increases and the heart is thereby enlarged. In view of this, another method for assessing whether the dry weight is appropriate or not is to make an assessment based on the size of a heart relative to the rib cage (cardiothoracic ratio) in a chest X-ray radiograph. When a cardiothoracic ratio in a chest X-ray radiograph taken after hemodialysis is approximately 50%, the extracellular fluid volume is determined as appropriate (Gunal AI: How to determine 'dry weight'? Kidney Int 3: 377-379, 2013). Further, it is known that atrial natriuretic peptide concentration (which will be referred to as hANP concentration, hereinbelow) is secreted in large quantity when the heart is strained. In view of this, an hANP concentration in post-hemodialysis blood is measured, and when it is an appropriate concentration (40 to 60 pg/mL), the dry weight is determined as appropriate (Eriko ISHII, et al.: The target range of plasma ANP level for dry weight adjustment in HD patients, Journal of Japanese Society for Dialysis Therapy, 37:1417-1422, 2004). However, in patients with cardiac failure and/or cardiac valvular disease, the hANP concentration and/or the cardiothoracic ratio increase(s) even though the extracellular fluid volume is appropriate (Brandt RR' et al: Atrial natriuretic peptide in heart failure. J Am Coll Cardiol. 22 (4 Suppl A): 86A-92A, 1993). Here, hemodialysis patients have a high probability of getting cardiac failure and/or cardiac valvular disease.

Summary of Invention

Technical Problem

In a hemodialysis patient, water has excessively accumulated in extracellular compartment before hemodialysis. Thus, the water is removed during hemodialysis until the extracellular fluid volume of the patient becomes equal to that of a person with normally functioning kidneys. That is, the water is removed during hemodialysis until the patient's weight becomes his/her dry weight.

The extracellular fluid volume, based on which the dry weight is determined, is estimated based on clinical symptoms. However, evaluation results based on the clinical symptoms may often be incorrect. Thus, even though the water is removed during hemodialysis until the patient's weight becomes equal to the dry weight, the post-hemodialysis extracellular fluid volume may not always be equal to the extracellular fluid volume of a person with normally functioning kidneys, actually.

Further, fat amount and/or muscle mass of a hemodialysis patient change depending on his/her nutritional condition. The patient's extracellular fluid volume changes as the fat amount and/or muscle mass change. Thus, the dry weight needs to be updated regularly. However, conventional methods for determining dry weight have accuracy issues and are not suitable for frequently resetting dry weight to keep the extracellular fluid volume at appropriate level. For example, whether edema is present or not is determined based on the skin resilience level, however, for the elderly who are usually inferior in skin resilience, it is hard to determine whether edema is present or not based on the skin resilience level. That is, it is hard to assess the extracellular fluid volume of the elderly based on whether edema is present or not. Further, since edema does not occur unless the extracellular fluid volume is greater by at least 3 to 5 kg than the appropriate extracellular fluid volume (Gunal AI: How to determine 'dry weight'? Kidney Int 3: 377-379, 2013), the method based on whether edema is observed or not can detect abnormality in the extracellular fluid volume only when the extracellular fluid volume is significantly increased. Furthermore, the assessment may differ depending on skills of assessors (e.g., doctors), thus it cannot be said that whether the extracellular fluid volume is deficient or excessive is correctly determined based on clinical symptoms. Another method for assessing an extracellular fluid volume based on a chest X-ray radiograph requires time and labor to take a chest X-ray radiograph and also requires a facility for taking X-ray radiographs. Further, a hemodialysis patient with cardiac depression, that is, a hemodialysis patient with cardiac failure and/or cardiac valvular disease, may have a large cardiothoracic ratio even though the dry weight is appropriate, that is, even though the extracellular fluid volume is appropriate. In other words, the cardiothoracic ratio is not reliable when the patient has cardiac failure and/or cardiac valvular disease. Another method for assessing an extracellular fluid volume from an hANP concentration costs significantly for measuring an hANP concentration and is not suitable to be frequently carried out. Further, a hemodialysis patient with cardiac depression, that is, a hemodialysis patient with cardiac failure and/or cardiac valvular disease, may have a high hANP concentration even though the extracellular fluid volume is appropriate. Thus, for the hemodialysis patient with a cardiac disease, whether his/her extracellular fluid volume is appropriate or not cannot be accurately determined based on chest X-ray radiograph or hANP concentration.

The disclosure herein discloses a technique that assesses an extracellular fluid volume of a hemodialysis patient accurately and easily.


Solution to Technical Problem


An extracellular fluid volume calculator disclosed herein may comprise: an acquirement unit configured to acquire a membrane area of a dialyzer used for hemodialysis; and a processor configured to calculate a post-hemodialysis extracellular fluid volume based on a difference between a pre-hemodialysis amount of uric acid and a post-hemodialysis amount of uric acid. The processor may be configured to: calculate a removal amount of uric acid removed by hemodialysis based on a dialyzer overall mass transfer-area coefficient for uric acid; and calculate the dialyzer overall mass transfer-area coefficient for uric acid based on the membrane area of the dialyzer acquired by the acquirement unit.

The above-described extracellular fluid volume calculator calculates the post-hemodialysis extracellular fluid volume based on the difference between the pre-hemodialysis amount of uric acid and the post-hemodialysis amount of uric acid in extracellular compartment. That is, the extracellular fluid volume calculator can calculate the post-hemodialysis extracellular fluid volume based on a theory that the removal amount of uric acid removed by hemodialysis is equal to the difference between the pre-hemodialysis amount of uric acid in the extracellular compartment and the post-hemodialysis amount of uric acid in the extracellular compartment. It is known that the removal amount of uric acid can be calculated from a dialyzer clearance for uric acid during hemodialysis and a plasma uric acid concentration. The dialyzer clearance for uric acid during hemodialysis can be calculated, using a publicly known formula, from a plasma flow rate and a dialysate flow rate passing through the dialyzer and a dialyzer overall mass transfer-area coefficient for uric acid. The dialyzer overall mass transfer-area coefficient for uric acid can be calculated, using a publicly known formula, from a dialyzer clearance for uric acid that is measured with a specific plasma flow rate and a specific dialysate flow rate in an ex-vivo experiment using bovine blood. Usually, for this kind of experiment, the plasma flow rate passing through the dialyzer is set at 136 mL/min and the dialysate flow rate passing through the dialyzer is set at 500 mL/min. However, it is not practical to carry out, for all dialyzers to be used for hemodialysis, such ex-vivo experiments to calculate their overall mass transfer-area coefficients for uric acid. As a result of dedicated study of the inventors, it has been revealed that the dialyzer overall mass transfer-area coefficient for uric acid is correlated with the membrane area of the dialyzer. Thus, the dialyzer overall mass transfer-area coefficient for uric acid can be calculated from the membrane area of the dialyzer. This means that the dialyzer clearance for uric acid can be calculated from the membrane area of the dialyzer. Therefore, the removal amount of uric acid can be easily calculated by using the membrane area of the dialyzer, thereby significantly facilitating calculation of the extracellular fluid volume.

A method for calculating an extracellular fluid volume disclosed herein may comprise: an acquirement step of acquiring a membrane area of a dialyzer used for hemodialysis; and a calculation step of calculating a post-hemodialysis extracellular fluid volume based on a difference between a pre-hemodialysis amount of uric acid and a post-hemodialysis amount of uric acid. The calculation step may comprise: a first calculation step of calculating a dialyzer overall mass transfer-area coefficient for uric acid based on the membrane area of the dialyzer acquired in the acquirement step; and a second calculation step of calculating a removal amount of uric acid removed by hemodialysis based on the dialyzer overall mass transfer-area coefficient for uric acid calculated in the first calculation step.

According to the above-described method for calculating the extracellular fluid volume, in the calculation step of calculating the post-hemodialysis extracellular fluid volume based on the difference between the pre-hemodialysis amount of uric acid and the post-hemodialysis amount of uric acid, the dialyzer overall mass transfer-area coefficient for uric acid is calculated based on the membrane area of the dialyzer, and the removal amount of uric acid is calculated based on the calculated dialyzer overall mass transfer-area coefficient for uric acid. Thus, this method can bring the same effects as those of the above-described extracellular fluid volume calculator.

Brief Description of Drawings

FIG. 1 shows a system configuration of an extracellular fluid volume calculator according to an embodiment.
FIG. 2 schematically shows substances in extracellular and intracellular compartments.
FIG. 3 shows a relationship between membrane areas of dialyzers and the dialyzer overall mass transfer-area coefficients for uric acid.
FIG. 4 shows measured removal amounts of uric acid actually removed by hemodialysis and removal amounts of uric acid calculated from membrane areas of dialyzers.
FIG. 5 shows a flowchart for one example of process a processor follows to calculate an extracellular fluid volume.

Description of Embodiments

Some of the features characteristic to below-described embodiments will herein be listed. It should be noted that the respective technical elements are independent of one another, and are useful solely or in combinations. The combinations thereof are not limited to those described in the claims as originally filed.

(Feature 1) In the extracellular fluid volume calculator disclosed herein, the acquirement unit may be configured to acquire a plasma flow rate passing through the dialyzer and a dialysate flow rate passing through the dialyzer. The processor may be configured to: calculate the removal amount of uric acid based on a dialyzer clearance for uric acid; and calculate the dialyzer clearance for uric acid based on the plasma flow rate and the dialysate flow rate acquired by the acquirement unit and the calculated dialyzer overall mass transfer-area coefficient for uric acid. This configuration enables the elements used for calculation of the removal amount of uric acid to be acquired with few additional, special procedure. Thus, the extracellular fluid volume calculator facilitates the calculation of the removal amount of uric acid, thereby facilitating the calculation of the extracellular fluid volume.

Embodiment

Hereinbelow, an extracellular fluid volume calculator 10 according to an embodiment will be described. The extracellular fluid volume calculator 10 is configured to calculate an extracellular fluid volume in the body of a hemodialysis patient. It is known that even when a water volume in the hemodialysis patient's body becomes excessive (i.e., the patient becomes overhydrated) or becomes insufficient (i.e., the patient becomes underhydrated), a water volume in an intracellular compartment 40 hardly changes and only a water volume in an extracellular compartment 50 changes. Thus, it is an extracellular fluid volume that is needed to be adjusted by water removal through hemodialysis. In order to assess whether a post-hemodialysis extracellular fluid volume of the hemodialysis patient is appropriate or not, the extracellular fluid volume calculator 10 according to the present embodiment is configured to calculate the post-hemodialysis extracellular fluid volume of the hemodialysis patient.

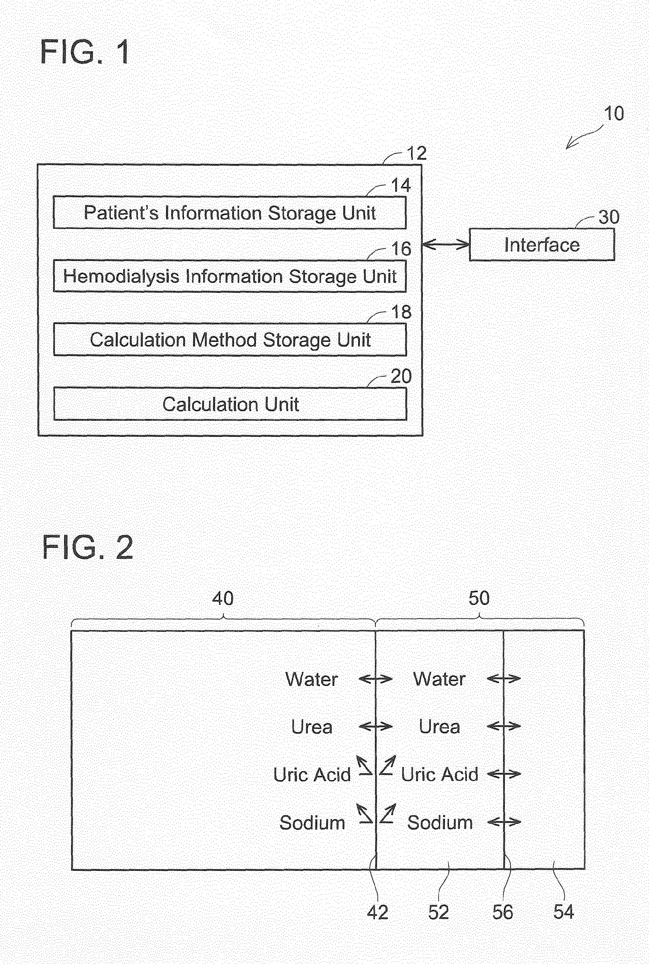

As shown in FIG. 1, the extracellular fluid volume calculator 10 includes a processor 12 and an interface 30. The processor 12 may be configured of a computer including a CPU, a ROM, a RAM, etc., for example. The processor 12 functions as a calculation unit 20 shown in FIG. 1 by the computer executing a program. A process executed by the calculation unit 20 will be described later in detail.

As shown in FIG. 1, the processor 12 includes a patient's information storage unit 14, a hemodialysis information storage unit 16, and a calculation method storage unit 18. The patient's information storage unit 14 is configured to store various kinds of information about the hemodialysis patient. The patient's information storage unit 14 stores information about the hemodialysis patient inputted through the interface 30 and information about the hemodialysis patient calculated by the calculation unit 20. The information about the hemodialysis patient inputted through the interface 30 includes pre-hemodialysis and post-hemodialysis plasma uric acid concentrations, and pre-hemodialysis and post-hemodialysis hematocrit values of the hemodialysis patient, for example. The information about the hemodialysis patient calculated by the calculation unit 20 includes a post-hemodialysis extracellular fluid volume calculated based on the information inputted through the interface 30, for example.

The hemodialysis information storage unit 16 is configured to store various types of information about hemodialysis. The hemodialysis information storage unit 16 stores information about hemodialysis inputted through the interface 30 and information about hemodialysis calculated by the calculation unit 20. The information about hemodialysis inputted through the interface 30 includes a removal volume of water by hemodialysis, a hemodialysis period, blood and dialysate flow rates passing through a dialyzer during hemodialysis, and the membrane area of a dialyzer used in hemodialysis, for example. The information about hemodialysis calculated by the calculation unit 20 includes an overall mass transfer-area coefficient for uric acid of a dialyzer used in hemodialysis, a dialyzer clearance for uric acid, and a removal amount of uric acid that are calculated based on the information inputted through the interface 30, for example.

The calculation method storage unit 18 is configured to store various mathematical formulas used for calculating the post-hemodialysis extracellular fluid volume. For example, the calculation method storage unit 18 stores formulas of Mathematical 3, 6 to 9, 13, 15, and 23 which will be described later in detail. The calculation unit 20 is configured to calculate various numerical values that are used for calculating the post-hemodialysis extracellular fluid volume by substituting the various numerical values stored in the patient's information storage unit 14 and the hemodialysis information storage unit 16 into the formulas stored in the calculation method storage unit 18.

The interface 30 is a display device configured to provide (output) various types of information calculated by the extracellular fluid volume calculator 10 to an operator, and is also an input device configured to receive instructions and information from the operator. The interface 30 can display, to the operator, a calculated post-hemodialysis extracellular fluid volume and the like, for example. Further, the interface 30 can receive input of various types of information about the hemodialysis patient (pre-hemodialysis and post-hemodialysis plasma uric acid concentrations, pre-hemodialysis and post-hemodialysis hematocrit values, etc.) and various types of information about hemodialysis (removal volume of water, hemodialysis period, blood flow rate passing through a dialyzer, dialysate flow rate passing through a dialyzer, membrane area of a dialyzer used in hemodialysis, etc.).

Here, a method for calculating a post-hemodialysis extracellular fluid volume using various types of information inputted to the interface 30 will be described. The extracellular fluid volume calculator 10 according to the present embodiment is configured to calculate a post-hemodialysis extracellular fluid volume, focusing on the difference between pre-hemodialysis and post-hemodialysis uric acid quantities in the extracellular compartment 50. As shown in FIG. 2, a fluid compartment in a body is divided into the intracellular compartment 40 and the extracellular compartment 50, and the extracellular compartment 50 is divided into an interstitial compartment 52 and an intravascular compartment 54. Uric acid is distributed in both the intracellular compartment 40 and the extracellular compartment 50, but does not pass through a cell membrane 42 within four hours or so, which is a typical hemodialysis period. On the other hand, uric acid passes through a capillary membrane 56. Therefore, the post-hemodialysis extracellular fluid volume can be calculated by focusing on the difference between pre-hemodialysis and post-hemodialysis uric acid quantities in the extracellular compartment 50.

The method for calculating a post-hemodialysis extracellular fluid volume based on the difference between pre-hemodialysis and post-hemodialysis uric acid quantities in the extracellular compartment 50 will be described further in detail. An amount of uric acid removed from the extracellular compartment 50 by hemodialysis (which may be referred to as "removal amount of uric acid", hereinbelow) is equal to a difference between an amount of uric acid distributed in the extracellular compartment 50 before hemodialysis and an amount of uric acid distributed in the extracellular compartment 50 after hemodialysis. Here, an amount of uric acid distributed in the extracellular compartment 50 can be calculated by multiplying an extracellular fluid volume by a uric acid concentration in the extracellular compartment 50. Thus, the formula of Mathematical 1 below holds up. Here, $_{acid}E$ represents removal amount of uric acid, $_{ecf}Vs$ represents pre-hemodialysis extracellular fluid volume, $_{ecf}Ve$ represents post-hemodialysis extracellular fluid volume, $_{acid}Cs$ represents pre-hemodialysis uric acid concentration in the extracellular compartment 50, and $_{acid}Ce$ represents post-hemodialysis uric acid concentration in the extracellular compartment 50. Since uric acid passes through the capillary membrane 56, a uric acid concentration in the intravascular compartment 54 is equal to a uric acid concentration in the interstitial compartment 52. Further, since the extracellular compartment 50 is the combination of the intravascular compartment 54 and the interstitial compartment 52, the uric acid concentration in the extracellular compartment 50 can be considered as the uric acid concentration in the intravascular compartment 54. Thus, the uric acid concentration in the extracellular compartment 50 can be considered as a plasma uric acid concentration.

[Mathematical 1]

$$_{acid}E = {}_{ecf}Vs \times {}_{acid}Cs - {}_{ecf}Ve \times {}_{acid}Ce$$

Next, a volume of water removed from the extracellular compartment 50 by hemodialysis will be described. A difference between a pre-hemodialysis extracellular fluid volume and a post-hemodialysis extracellular fluid volume is equal to a volume of water removed from the body by hemodialysis (which may be referred to as "removal volume of water", hereinbelow). Therefore, the formula of Mathematical 2 below holds up, where $_{dial}EW$ represents removal volume of water.

[Mathematical 2]

$$_{ecf}Vs - {_{ecf}}Ve = {_{dial}}EW$$

From the formulas of Mathematical 1 and 2, the formula of Mathematical 3 below is obtained.

[Mathematical 3]

$$_{ecf}Ve = \frac{_{acid}E - {_{dial}}EW \times {_{acid}}Cs}{_{acid}Cs - {_{acid}}Ce}$$

As described, the pre-hemodialysis uric acid concentration $_{acid}Cs$ in the extracellular compartment 50 is equal to the pre-hemodialysis plasma uric acid concentration, and the post-hemodialysis uric acid concentration $_{acid}Ce$ in the extracellular compartment 50 is equal to the post-hemodialysis plasma uric acid concentration. Further, the pre-hemodialysis uric acid concentration $_{acid}Cs$ and the post-hemodialysis uric acid concentration $_{acid}Ce$ can be acquired as measured values. Furthermore, the removal volume of water $_{dial}EW$ can also be acquired as a measured value. Thus, post-hemodialysis extracellular fluid volume $_{ecf}Ve$ can be calculated using the above formula of Mathematical 3 by acquiring or calculating a removal amount of uric acid $_{acid}E$. A method for calculating the removal amount of uric acid $_{acid}E$ will be described below.

In the present embodiment, a dialyzer clearance for uric acid is necessary to calculate the removal amount of uric acid $_{acid}E$, and a dialyzer overall mass transfer-area coefficient for uric acid is necessary to calculate the dialyzer clearance for uric acid. In the present embodiment, an overall mass transfer-area coefficient for uric acid of a dialyzer used in hemodialysis is calculated based on the membrane area of the dialyzer. Specifically, the dialyzer overall mass transfer-area coefficient for uric acid is calculated based on the membrane area of the dialyzer used in hemodialysis, the dialyzer clearance for uric acid is calculated from the calculated dialyzer overall mass transfer-area coefficient for uric acid, a blood flow rate passing through the dialyzer, and a dialysate flow rate passing through the dialyzer, and then the removal amount of uric acid $_{acid}E$ is calculated using the calculated dialyzer clearance for uric acid.

Other than the method according to the present embodiment, the removal amount of uric acid $_{acid}E$ may be acquired by measuring an amount of uric acid removed into the dialysate by hemodialysis. Specifically, it can be calculated by measuring a uric acid concentration in the used dialysate after hemodialysis and by multiplying the measured uric acid concentration by the volume of the used dialysate. When this method is used for a patient with a low plasma uric acid concentration, however, the accuracy of the calculated removal amount of uric acid may be low due to measurement error because a uric acid concentration in the used dialysate of such patient is extremely low. Generally, the plasma uric acid concentration at the end of hemodialysis is approximately 2 mg/dL, and in this case, the uric acid concentration in the used dialysate is approximately 0.8 mg/dL. Meanwhile, in some patients, the plasma uric acid concentration at the end of hemodialysis is approximately 1.0 mg/dL, and in this case, the uric acid concentration in the used dialysate can often be lower than 0.5 mg/dL. In case where a uric acid concentration in an analyte is measured as a measured value using a typical measurement instrument, the measured uric acid concentration in the analyte is indicated only up to the first digit after decimal point, such as 4.5 mg/dL. If the uric acid concentration in the used dialysate is erroneously measured as 0.4 mg/dL due to measurement error despite that it actually is approximately 0.5 mg/dL, the error is approximately 20%. If the removal amount of uric acid is calculated based thereon, it also includes the great error of approximately 20%. In view of this, in the present embodiment, the removal amount of uric acid $_{acid}E$ is calculated based on the plasma uric acid concentration and the clearance for uric acid of a dialyzer used in hemodialysis in order to accurately acquire the removal amount of uric acid $_{acid}E$ even for patients with low plasma uric acid concentration.

A method for calculating a dialyzer clearance for uric acid according to the present embodiment will be described in detail below. It is known that the plasma uric acid concentration decreases exponentially during hemodialysis. Thus, the formula of Mathematical 4 below holds up, where $_{acid}C(t)$ represents plasma uric acid concentration at a time t during hemodialysis, and $_{acid}A$ represents coefficient. The coefficient $_{acid}A$ is expressed as in the formula of Mathematical 5 below, where Td is a hemodialysis period.

[Mathematical 4]

$$_{acid}C(t) = {_{acid}Cs} \times \exp({_{acid}A} \times t)$$

[Mathematical 5]

$$_{acid}A = \frac{\ln({_{acid}Ce}/{_{acid}Cs})}{Td}$$

The formula of Mathematical 6 below can be obtained from the formulas of Mathematical 4 and 5.

[Mathematical 6]

$$_{acid}C(t) = {_{acid}Cs} \times ({_{acid}Ce}/{_{acid}Cs})^{\wedge}\left( \frac{t}{Td} \right)$$

A uric acid removal rate at the time t during hemodialysis can be calculated by multiplying the dialyzer clearance for uric acid at the time t by the plasma uric acid concentration $_{acid}C(t)$ at the time t. Thus, the formula of Mathematical 7 below holds up, where $_{acid}F(t)$ represents uric acid removal rate at the time t during hemodialysis and $_{acid}K(t)$ represents dialyzer clearance for uric acid at the time t during hemodialysis.

[Mathematical 7]

$$_{acid}F(t) = {_{acid}K(t)} \times {_{acid}C(t)}$$

The dialyzer clearance $_{acid}K(t)$ for uric acid is a variable depending on the time t. Uric acid is distributed in both plasma and blood cells (Nagendra S, et al: A comparative study of plasma uric acid, erythrocyte uric acid and urine uric acid levels in type 2 diabetic subjects. Merit Research Journal 3: 571-574, 2015), however, it does not pass through red blood cell membranes, which are cell membranes. Thus, uric acid is removed only from a plasma compartment of blood passing through the dialyzer during hemodialysis (Eric Descombes, et al: Diffusion kinetics of urea, creatinine and uric acid in blood during hemodialysis. Clinical implications. Clinical Nephrology 40: 286-295, 1993). Accordingly, not the blood flow rate passing through the dialyzer but a plasma flow rate passing through the dialyzer is used to calculate the dialyzer clearance $_{acid}K(t)$ for uric acid. By the way, as the blood is concentrated by the water removal during hemodialysis, the hematocrit value increases over time during hemodialysis. Thus, the plasma flow rate is not constant during hemodialysis. Accordingly, the dialyzer clearance $_{acid}K(t)$ for uric acid, which is calculated using the plasma flow rate passing through the dialyzer, also changes with the change in the plasma flow rate passing through the dialyzer over time. That is, the dialyzer clearance $_{acid}K(t)$ for uric acid is a variable depending on the time t. To calculate the removal amount of uric acid $_{acid}E$, the uric acid removal rate at the time t calculated using the formula of Mathematical 7 is added up while changing the time t from t=0 up to t=Td at regular intervals (e.g., at intervals of 0.1 min.). That is, the removal amount of uric acid $_{acid}E$ is calculated using the formula of Mathematical 8 below.

**[Mathematical 8]**

$$_{acid}E = \sum_{t=0}^{Td} \{_{acid}K(t) \times {}_{acid}C(t)\}$$

The dialyzer clearance $_{acid}K(t)$ for uric acid at the time t can be calculated using a known formula for calculating a dialyzer clearance for solute. That is, the dialyzer clearance $_{acid}K(t)$ for uric acid at the time t can be calculated using the formula of Mathematical 9 below, where $_{acid}KoA$ represents dialyzer overall mass transfer-area coefficient for uric acid, $Qp_t$ represents plasma flow rate passing through the dialyzer at the time t during hemodialysis, and $Q_D$ represents dialysate flow rate passing through the dialyzer.

**[Mathematical 9]**

$$_{acid}K(t) = \frac{1 - \exp\{_{acid}KoA(\dfrac{1}{Q_{Pt}} - \dfrac{1}{Q_D})\}}{\dfrac{1}{Q_D} - \dfrac{1}{Q_{Pt}} \exp\{_{acid}KoA(\dfrac{1}{Q_{Pt}} - \dfrac{1}{Q_D})\}}$$

The dialysate flow rate $Q_D$ passing through the dialyzer can be acquired as a measured value. Thus, the dialyzer clearance $_{acid}K(t)$ for uric acid at the time t can be calculated by acquiring or calculating the dialyzer overall mass transfer-area coefficient $_{acid}KoA$ for uric acid and the plasma flow rate $Qp_t$ passing through the dialyzer at the time t.

A method for calculating the dialyzer overall mass transfer-area coefficient $_{acid}KoA$ for uric acid will be described. As a result of dedicated study by the inventors, it has been revealed that, in case where a hollow fiber dialyzer is used, the dialyzer overall mass transfer-area coefficient $_{acid}KoA$ for uric acid is correlated with a membrane area of the dialyzer. A relationship between the dialyzer overall mass transfer-area coefficient $_{acid}KoA$ for uric acid and the membrane area of the dialyzer will be described below.

An example in which overall mass transfer-area coefficients for uric acid were respectively calculated for dialyzers with different membrane areas in an ex-vivo experiment using bovine blood will be explained. Four different hollow fiber dialyzers, which belong to the same series (PES-SEaeo series manufactured by Nipro Corporation), were used in the experiment, and their membrane areas were 1.1 m2, 1.5 m2, 2.1 m2, and 2.5 m2. Bovine blood with hematocrit value of 32% was flowed through each of the dialyzers at 200 mL/min, and dialysate was also flowed therethrough at 500 mL/min in the opposite direction to the flowing direction of the bovine blood. When one minute elapsed since the bovine blood (which may be referred to simply as "blood" hereinbelow) started being flowed through the dialyzers, the blood was sampled at a blood inlet and a blood outlet of each dialyzer. The blood samples were subjected to centrifugal process immediately and uric acid concentrations in the plasma were measured. Based on these measured values, ex-vivo clearance for uric acid of each dialyzer was calculated using the formula of Mathematical 10 below, where K represents dialyzer clearance for uric acid, Cin represents plasma uric acid concentration at the blood inlet of the dialyzer, Cout represents plasma uric acid concentration at the blood outlet of the dialyzer, and QP represents plasma flow rate passing through the dialyzer.

**[Mathematical 10]**

$$K = \frac{Cin - Cout}{Cin} \times QP$$

Plasma means a part of blood from which blood cell components are excluded. Thus, the plasma flow rate QP passing through the dialyzer in the formula of Mathematical 10 can be calculated using the formula of Mathematical 11 below, where Ht represents hematocrit value and $Q_B$ represents blood flow rate passing through the dialyzer.

[Mathematical 11]

$$QP = (1 - Ht/100) \times Q_B$$

As mentioned, the hematocrit value of the bovine blood used in the above ex-vivo experiment was 32%, and the blood flow rate $Q_B$ passing through the dialyzers was 200 mL/min. By substituting these values into Mathematical 11, 136 mL/min was obtained as the plasma flow rate QP passing through the dialyzers used in the experiment.

Next, an overall mass transfer-area coefficient for uric acid was calculated for each of the dialyzers using the formula of Mathematical 12 below, which is a modification of the formula of Mathematical 9. In the formula of Mathematical 12, KoA represents dialyzer overall mass transfer-area coefficient for uric acid, $Q_D$ represents dialysate flow rate passing through the dialyzer in the ex-vivo experiment, and K represents dialyzer clearance for uric acid measured in the ex-vivo experiment. As mentioned, in the ex-vivo experiment, the dialysate flow rate $Q_D$ passing through the dialyzers was 500 mL/min, and the plasma flow rate QP passing through the dialyzers was 136 mL/min.

[Mathematical 12]

$$KoA = \frac{\ln\left(\dfrac{1 - K/Q_D}{1 - K/QP}\right)}{1/QP - 1/Q_D}$$

As above, overall mass transfer-area coefficients for uric acid were calculated for the dialyzers. The result is shown below.

[Table 1]

| Membrane Area ($m^2$) | Clearance for Uric Acid (ml/min) | Overall Mass Transfer-Area Coefficient for Uric Acid (ml/min) |
|---|---|---|
| 1.1 | 111 | 187 |
| 1.5 | 122 | 220 |
| 2.1 | 130 | 248 |
| 2.5 | 129 | 247 |

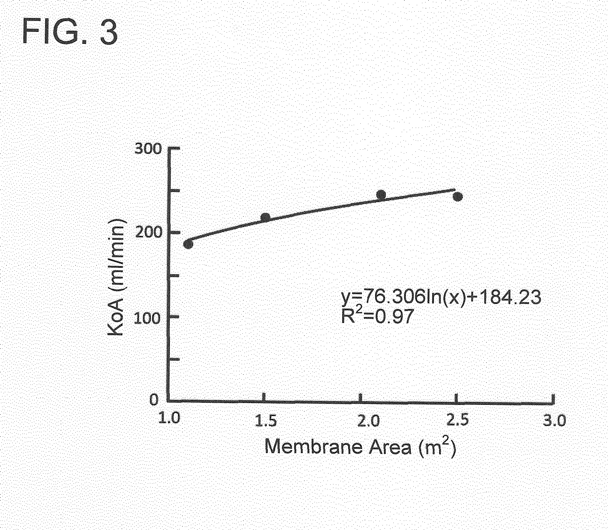

Regarding the result shown in Table 1, FIG. 3 shows a graph for a relationship between the membrane areas of the dialyzers and their overall mass transfer-area coefficients for uric acid. As shown in FIG. 3, it has been confirmed that there is a correlation relationship between the membrane areas of the dialyzers and their overall mass transfer-area coefficients for uric acid as expressed in the formula of Mathematical 13 below.

[Mathematical 13]

$$y = 76.306\ln(x) + 184.23$$

Accordingly, by substituting the membrane area of a dialyzer used in hemodialysis into the formula of Mathematical 13, the overall mass transfer-area coefficient KoA for uric acid of the dialyzer can be calculated.

Next, a method for calculating the plasma flow rate $Q_{pt}$ passing through the dialyzer at the time t during hemodialysis will be described. The volume of plasma compartment in blood of a patient is expressed in the formula of Mathematical 14 below, where PV represents plasma volume, BV represents blood volume, and Ht represents hematocrit value.

[Mathematical 14]

$$PV = \{1-Ht/100\} \times BV$$

The formula of Mathematical 15 is obtained by rearranging the formula of Mathematical 4 into a relationship between the blood flow rate and the plasma flow rate passing through the dialyzer at the time t. Here, Ht(t) represents hematocrit value (%) at the time t during hemodialysis, $Q_B$ represents blood flow rate passing through the dialyzer at the time t during hemodialysis, and $Q_{Pt}$ represents plasma flow rate passing through the dialyzer at the time t during hemodialysis. The blood flow rate $Q_B$ passing through the dialyzer is constant during hemodialysis.

[Mathematical 15]

$$Q_{Pt} = \{1-Ht(t)/100\} \times Q_B$$

The blood flow rate $Q_B$ passing through the dialyzer does not change over time and can be acquired as a measured value. Thus, the plasma flow rate $Q_{Pt}$ passing through the dialyzer can be calculated by calculating the hematocrit value Ht(t) at the time t. Hereinbelow, a method for calculating the hematocrit value Ht(t) at the time t will be described.

With constant water removal rate, the blood volume in the body decreases substantially in a linear fashion during hemodialysis. This has been confirmed by the inventors studying measurement result from a BV meter (blood volume meter). Thus, a blood volume BV(t) at the time t during hemodialysis is expressed as in the formula of Mathematical 16, where BVs represents blood volume at t=0. Here, $\alpha$ is usually a negative value.

[Mathematical 16]

$$BV(t) = \alpha \times t + BVs$$

In Mathematical 17, the value $\alpha$ can be calculated by substituting t=Td in the formula of Mathematical 16, where BVe represents blood volume at the end of hemodialysis.

[Mathematical 17]

$$BVe = \alpha \times Td + BVs$$

The formula of Mathematical 18 below is obtained by rearranging the above formula of Mathematical 17.

[Mathematical 18]

$$\alpha = \frac{BVe - BVs}{Td}$$

The formula of Mathematical 19 below is obtained by substituting the value $\alpha$ calculated in the formula of Mathematical 18 into the above formula of Mathematical 16.

[Mathematical 19]

$$BV(t) = \frac{BVe - BVs}{Td} \times t + BVs$$

The total number of red blood cells in the body is constant and does not change over time. This means that the total red blood cell volume is also constant and does not change over time. The total red blood cell volume in the body is calculated by multiplying the blood volume in the body by one-hundredth of the hematocrit value. Thus, the formulas of Mathematical 20 to 22 are obtained, where TE represents total red blood cell volume in the body.

[Mathematical 20]

$$BV(t) \times Ht(t)/100 = TE$$

[Mathematical 21]

$$BVs \times Hts/100 = TE$$

[Mathematical 22]

$$BVe \times Hte/100 = TE$$

The formula of Mathematical 23 below is obtained from the above formulas of Mathematical 19 to 22.

[Mathematical 23]

$$Ht(t) = \frac{Td}{(Hts/Hte - 1) \times t + Td} \times Hts$$

A pre-hemodialysis hematocrit value Hts and a post-hemodialysis hematocrit value Hte can be acquired as measured values. Further, as described, the hemodialysis period Td can be acquired as a measured value. Thus, the hematocrit value $Ht(t)$ at the time t during hemodialysis can be calculated by substituting the pre-hemodialysis hematocrit value Hts, the post-hemodialysis hematocrit value Hte, and the hemodialysis period Td into the formula of Mathematical 23. Then, the plasma flow rate $Q_{Pt}$ passing through the dialyzer at the time t can be calculated by substituting the hematocrit value $Ht(t)$ at the time t calculated in the formula of Mathematical 23 and the acquired blood flow rate $Q_B$ passing through the dialyzer into the formula of Mathematical 15. That is, the plasma flow rate $Q_{Pt}$ passing through the dialyzer at the time t can be calculated from the pre-hemodialysis hematocrit value Hts, the post-hemodialysis hematocrit value Hte, the hemodialysis period Td, and the blood flow rate $Q_B$.

The dialyzer clearance $_{acid}K(t)$ for uric acid at the time t can be calculated by substituting the dialyzer overall mass transfer-area coefficient $_{acid}KoA$ for uric acid calculated using the formula of Mathematical 13, the plasma flow rate $Q_{Pt}$ passing through the dialyzer at the time t calculated using the formula of Mathematical 15, and the dialysate flow rate $Q_D$ which is constant throughout the process into the formula of Mathematical 9. That is, the dialyzer clearance $_{acid}K(t)$ for uric acid at the time t can be calculated from the membrane area of the dialyzer used in hemodialysis, the pre-hemodialysis hematocrit value Hts, the post-hemodialysis hematocrit value Hte, the hemodialysis period Td, the blood flow rate $Q_B$ passing through the dialyzer, and the dialysate flow rate $Q_D$ which is constant throughout the process.

Further, the removal amount of uric acid $_{acid}E$ can be calculated by substituting the dialyzer clearance $_{acid}K(t)$

for uric acid at the time t calculated using the formula of Mathematical 9 and the plasma uric acid concentration $_{acid}C(t)$ at the time t calculated using the formula of Mathematical 6 into the formula of Mathematical 8. That is, the removal amount of uric acid $_{acid}E$ can be calculated from the membrane area of the dialyzer used in hemodialysis, the pre-hemodialysis plasma uric acid concentration $_{acid}Cs$, the post-hemodialysis plasma uric acid concentration $_{acid}Ce$, the pre-hemodialysis hematocrit value Hts, the post-hemodialysis hematocrit value Hte, the hemodialysis period Td, the blood flow rate $Q_B$ passing through the dialyzer, and the dialysate flow rate $Q_D$ passing through the dialyzer.

Further, the post-hemodialysis extracellular fluid volume $_{ecf}Ve$ can be calculated by substituting the removal amount of uric acid $_{acid}E$ calculated using the formula of Mathematical 8 and the acquired pre-hemodialysis plasma uric acid concentration $_{acid}Cs$, post-hemodialysis plasma uric acid concentration $_{acid}Ce$, and removal volume of water $_{dial}EW$ into the formula of Mathematical 3.

As above, in the present embodiment, the post-hemodialysis extracellular fluid volume $_{ecf}Ve$ can be calculated from the pre-hemodialysis plasma uric acid concentration $_{acid}Cs$, the post-hemodialysis plasma uric acid concentration $_{acid}Ce$, the pre-hemodialysis hematocrit value Hts, the post-hemodialysis hematocrit value Hte, the removal volume of water $_{dial}EW$, the hemodialysis period Td, the blood flow rate $Q_B$ passing through the dialyzer, the dialysate flow rate $Q_D$ passing through the dialyzer, and the membrane area of the dialyzer used in hemodialysis.

In verification by the inventors, it has been confirmed that the removal amount of uric acid $_{acid}E$ is accurately calculated based on the overall mass transfer-area coefficient $_{acid}KoA$ for uric acid calculated from the membrane area using the formula of Mathematical 13. In the verification, for each of five patents on whom a dialyzer with membrane area of 0.9 m$^2$ was used, six patients on whom dialyzers with membrane area of 1.5 m$^2$ were used, and four patients on whom dialyzers with membrane area of 2.1 m$^2$ were used, a measured value of the total removal amount of uric acid removed by hemodialysis was compared to the removal amount of uric acid $_{acid}E$ calculated based on the overall mass transfer-area coefficient $_{acid}KoA$ for uric acid calculated from the membrane area using the formula of Mathematical 13. More specifically, a dialyzer with membrane area of 0.9 m$^2$ (FB-90Pβ, manufactured by Nipro Corporation) was used on five patients, a dialyzer with membrane area of 1.5 m$^2$ (APS-15SA, manufactured by Asahi Kasei Medical Co., Ltd.) was used on two patients, a dialyzer with membrane area of 1.5 m$^2$ (PES-1SSeα, manufactured by Nipro Corporation) was used on three patients, a dialyzer with membrane area of 1.5 m$^2$ (NV-15X, manufactured by Toray Medical Company Limited) was used on one patient, a dialyzer with membrane area of 2.1 m$^2$ (FDX-210GW, manufactured by Nikkiso Co., Ltd.) was used on one patient, a dialyzer with membrane area of 2.1 m$^2$ (PES-21 Sαeco, manufactured by Nipro Corporation) was used on one patient, and a dialyzer with membrane area of 2.1 m$^2$ (NV-21X, manufactured by Toray Medical Company Limited) was used on two patients. For each of these fifteen patients on whom the dialyzers were used, a measured value of the total removal amount of uric acid removed by hemodialysis was acquired and the removal amount of uric acid $_{acid}E$ was calculated based on the overall mass transfer-area coefficient $_{acid}KoA$ for uric acid calculated from the membrane area using the formula of Mathematical 13. The result is shown in Table 2 below.

[Table 2]

| Patient No. | Dialyzer Name | Manufacturer of Dialyzer | Membrane Area (m$^2$) | Measured Removal Amount of Uric Acid (g) | Estimated Removal Amount of Uric Acid (g) |
|---|---|---|---|---|---|
| 1 | FB-90Pβ | Nipro | 0.9 | 1.02 | 0.95 |
| 2 | FB-90Pβ | Nipro | 0.9 | 0.88 | 0.93 |
| 3 | FB-90Pβ | Nipro | 0.9 | 0.85 | 0.85 |
| 4 | PB-90Pβ | Nipro | 0.9 | 1.00 | 1.05 |
| 5 | FB-90Pβ | Nipro | 0.9 | 0.50 | 0.45 |
| 6 | APS-15SA | Asahi Kasei Medical | 1.5 | 1.30 | 1.14 |
| 7 | APS-15SA | Asahi Kasei Medical | 1.5 | 0.80 | 0.85 |
| 8 | PES-15SEα | Nipro | 1.5 | 1.20 | 1.07 |
| 9 | PES-15SEα | Nipro | 1.5 | 1.10 | 0.95 |
| 10 | PES-15SEα | Nipro | 1.5 | 0.60 | 0.68 |

(continued)

| Patient No. | Dialyzer Name | Manufacturer of Dialyzer | Membrane Area (m$^2$) | Measured Removal Amount of Uric Acid (g) | Estimated Removal Amount of Uric Acid (g) |
|---|---|---|---|---|---|
| 11 | NV-15X | Toray Medical | 1.5 | 0.80 | 0.84 |
| 12 | FDX-210GW | Nikkiso | 2.1 | 1.00 | 0.97 |
| 13 | PES-21 Saeco | Nipro | 2.1 | 1.21 | 1.26 |
| 14 | NV-21X | Toray Medical | 2.1 | 1.02 | 0.97 |
| 15 | NV-21 X | Toray Medical | 2.1 | 0.96 | 0.97 |

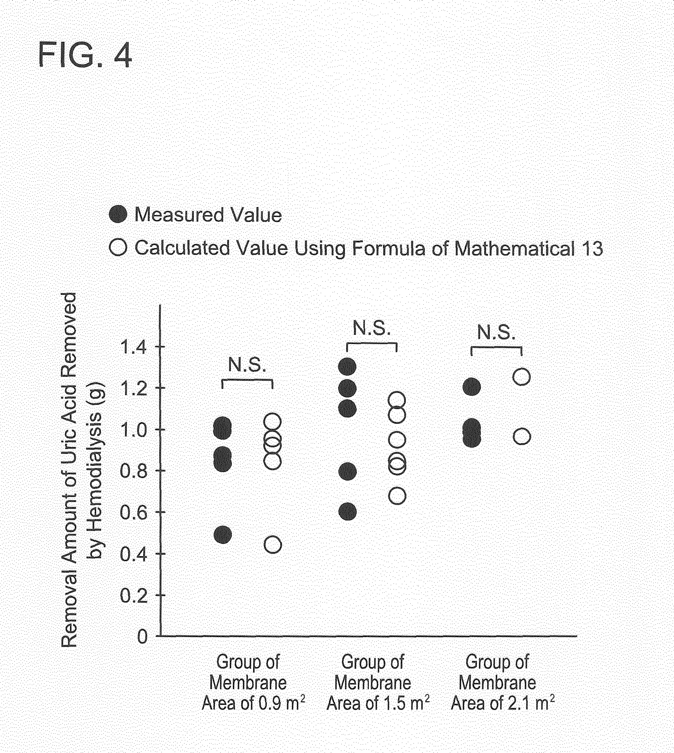

Regarding the result shown in Table 2, FIG. 4 shows a graph for the removal amounts of uric acid by hemodialysis by the dialyzer's membrane area. As shown in FIG. 4, each of the dialyzers did not produce a significant difference between the measured value and the calculated value.

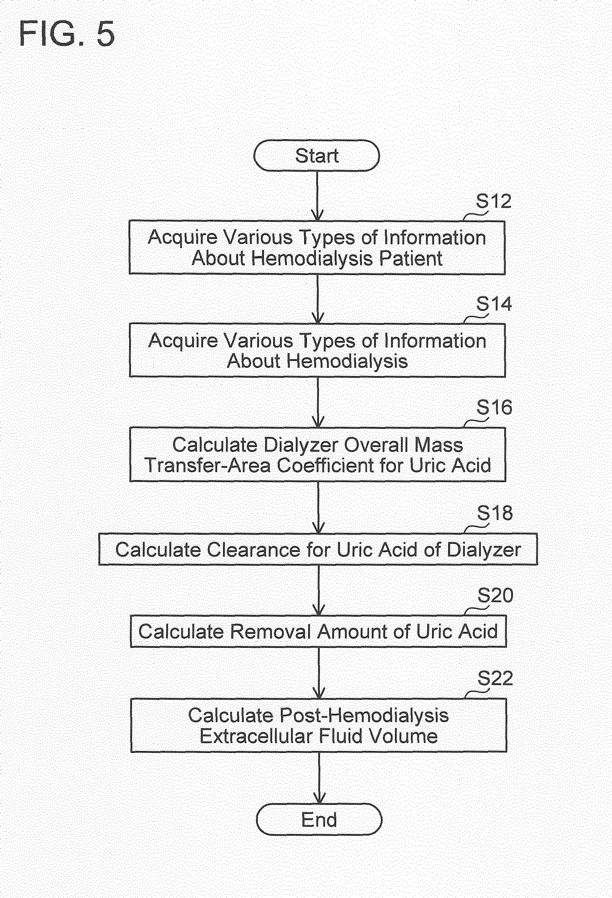

Next, a process executed by the extracellular fluid volume calculator 10 to calculate the post-hemodialysis extracellular fluid volume $_{ecf}$Ve will be described. As shown in FIG. 5, the processor 12 firstly acquires various types of information about the hemodialysis patient (S12). The various types of information about the hemodialysis patient include, for example, the pre-hemodialysis and post-hemodialysis plasma uric acid concentrations $_{acid}$Cs and $_{acid}$Ce, the pre-hemodialysis and post-hemodialysis hematocrit values Hts and Hte, and the like. The various types of information about the hemodialysis patient are acquired as described below, for example. How the pre-hemodialysis and post-hemodialysis plasma uric acid concentrations $_{acid}$Cs and $_{acid}$Ce are acquired will be described as an example. Firstly, blood samples are collected from the hemodialysis patient before and after hemodialysis. Then, the blood sample collected before hemodialysis is subjected to centrifugal process to separate the blood into blood cells and plasma, and the uric acid concentration $_{acid}$Cs in the separated plasma is measured, and further, the blood sample collected after hemodialysis is subjected to centrifugal process to separate the blood into blood cells and plasma, and the uric acid concentration $_{acid}$Ce in the separated plasma is measured. How the uric acid concentrations are measured is not particularly limited. The operator inputs the measured pre-hemodialysis and post-hemodialysis plasma uric acid concentrations $_{acid}$Cs and $_{acid}$Ce into the interface 30. The inputted pre-hemodialysis and post-hemodialysis plasma uric acid concentrations $_{acid}$Cs and $_{acid}$Ce are outputted from the interface 30 to the processor 12 and stored in the patient's information storage unit 14. Further, the pre-hemodialysis and post-hemodialysis hematocrit values Hts and Hte are respectively measured from the blood samples collected from the hemodialysis patient before and after hemodialysis. These measured values are stored in the patient's information storage unit 14 through the interface 30.

Next, the processor 12 acquires various types of information about hemodialysis (S14). The various types of information about hemodialysis include, for example, the removal volume of water $_{dial}$EW, the hemodialysis period Td, the blood flow rate $Q_B$ passing through the dialyzer, the dialysate flow rate $Q_D$ passing through the dialyzer, the membrane area of the dialyzer, and the like. The removal volume of water $_{dial}$EW, the hemodialysis period Td, the blood flow rate $Q_B$ passing through the dialyzer, the dialysate flow rate $Q_D$ passing through the dialyzer, and the membrane area of the dialyzer are inputted to the interface 30 by the operator. Then, the removal volume of water $_{dial}$EW, the hemodialysis period Td, the blood flow rate $Q_B$ passing through the dialyzer, the dialysate flow rate $Q_D$ passing through the dialyzer, and the membrane area of the dialyzer are outputted from the interface 30 to the processor 12 and stored in the hemodialysis information storage unit 16.

In the present embodiment, step S14 is carried out after step S12, however, no limitations are placed on the order of these steps. For example, step S14 may be carried out before step S12. Further, the acquisition order for the plural pieces of information acquired in step S12 and the plural pieces of information acquired in step S14 is not particularly limited. Any acquisition order may be applied as long as all of the items of step S12 and step S14 can be acquired. For example, the information to be acquired in step S14 may be acquired before all of the plural pieces of information to be acquired in step S12 are acquired.

Next, the calculation unit 20 calculates the dialyzer overall mass transfer-area coefficient $_{acid}$KoA for uric acid (S16), using the membrane area of the dialyzer used in hemodialysis among the information about hemodialysis acquired in step S14. The dialyzer overall mass transfer-area coefficient $_{acid}$KoA for uric acid is calculated using the formula of Mathematical 13 stored in the calculation method storage unit 18. Specifically, the calculation unit 20 substitutes the value of dialyzer's membrane area stored in the hemodialysis information storage unit 16 into the formula of Mathematical 13 to calculate the dialyzer overall mass transfer-area coefficient $_{acid}$KoA for uric acid. The calculated dialyzer overall mass transfer-area coefficient $_{acid}$KoA for uric acid is stored in the hemodialysis information storage unit 16.

Next, the calculation unit 20 calculates the clearance $_{acid}K(t)$ for uric acid of the dialyzer used in hemodialysis at the time t (S18), using the information about the hemodialysis patient acquired in step S12, the information about hemodialysis acquired in step S14, and the dialyzer overall mass transfer-area coefficient $_{acid}KoA$ for uric acid calculated in step 16. The clearance for uric acid of the dialyzer used in hemodialysis is calculated using the formulas of Mathematical 9, 15, and 23 stored in the calculation method storage unit 18.

Specifically, the calculation unit 20 firstly calculates the hematocrit value Ht(t) at the time t during hemodialysis by substituting the pre-hemodialysis hematocrit value Hts and the post-hemodialysis hematocrit value Hte stored in the patient's information storage unit 14 and the hemodialysis period Td stored in the hemodialysis information storage unit 16 into the formula of Mathematical 23. Then, the calculation unit 20 calculates the plasma flow rate $Q_{pt}$ passing through the dialyzer at the time t by substituting the hematocrit value Ht(t) at the time t calculated using the formula of Mathematical 23 and the blood flow rate $Q_B$ passing through the dialyzer stored in the hemodialysis information storage unit 16 into the formula of Mathematical 15. The calculation unit 20 then calculates the clearance $_{acid}K(t)$ for uric acid of the dialyzer used in hemodialysis at the time t by substituting the overall mass transfer-area coefficient $_{acid}KoA$ for uric acid calculated in step S16, the plasma flow rate $Q_{pt}$ passing through the dialyzer at the time t calculated using the formula of Mathematical 15, and the dialysate flow rate $Q_D$ passing through the dialyzer. The calculated clearance $_{acid}K(t)$ for uric acid at the time t is stored in the hemodialysis information storage unit 16.

Next, the calculation unit 20 calculates the removal amount of uric acid $_{acid}E$ (S20) using the information about the hemodialysis patient acquired in step S12, the information about hemodialysis acquired in step S14, and the calculated clearance $_{acid}K(t)$ for uric acid at the time t calculated in step S18. The removal amount of uric acid $_{acid}E$ is calculated using the formulas of Mathematical 6 and 8 stored in calculation method storage unit 18. Specifically, the calculation unit 20 firstly calculates the plasma uric acid concentration $_{acid}C(t)$ at the time t during hemodialysis by substituting the pre-hemodialysis plasma uric acid concentration $_{acid}Cs$ and the post-hemodialysis plasma uric acid concentration $_{acid}Ce$ stored in the patient's information storage unit 14 and the hemodialysis period Td stored in the hemodialysis information storage unit 16 into the formula of Mathematical 6. Then, the calculation unit 20 calculates the removal amount of uric acid $_{acid}E$ by substituting the clearance $_{acid}K(t)$ for uric acid at the time t calculated in step S18 and the plasma uric acid concentration $_{acid}C(t)$ at the time t during hemodialysis calculated using the formula of Mathematical 6 into the formula of Mathematical 8. The removal amount of uric acid $_{acid}E$ is stored in the hemodialysis information storage unit 16.

Lastly, the calculation unit 20 calculates the post-hemodialysis extracellular fluid volume $_{ecf}Ve$ (S22) using the information about the hemodialysis patient acquired in step S12, the information about hemodialysis acquired in step S14, and the removal amount of uric acid $_{acid}E$ calculated in step S20. The post-hemodialysis extracellular fluid volume $_{ecf}Ve$ is calculated using the formula of Mathematical 3 stored in the calculation method storage unit 18.

Specifically, the calculation unit 20 calculates the post-hemodialysis extracellular fluid volume $_{ecf}Ve$ by substituting the pre-hemodialysis uric acid concentration $_{acid}Cs$, the post-hemodialysis uric acid concentration $_{acid}Ce$, the removal volume of water $_{dial}EW$ stored in the hemodialysis information storage unit 16, and the removal amount of uric acid $_{acid}E$ calculated in step S20 into the formula of Mathematical 3. The calculated post-hemodialysis extracellular fluid volume $_{ecf}Ve$ is stored in the patient's information storage unit 14.

In the present embodiment, the post-hemodialysis extracellular fluid volume $_{ecf}Ve$ can be calculated focusing on the removal amount of uric acid $_{acid}E$ removed by hemodialysis. The removal amount of uric acid $_{acid}E$ can be calculated easily and accurately based on the clearance for uric acid of the dialyzer used in the hemodialysis. Further, the dialyzer clearance for solute can be calculated, using a known formula, based on the dialyzer overall mass transfer-area coefficient for the solute. By using the method according to the preset embodiment, the dialyzer overall mass transfer-area coefficient for the solute can be calculated from the membrane area of the dialyzer. That is, the present embodiment enables the post-hemodialysis extracellular fluid volume $_{ecf}Ve$ to be accurately calculated as a specific value from numerical values that can be easily acquired from the blood of a hemodialysis patient before and after hemodialysis and numerical values about hemodialysis that can be easily acquired.

In the present embodiment, the post-hemodialysis extracellular fluid volume $_{ecf}Ve$ is calculated without consideration for the volume of water that transfers from the extracellular compartment 50 to the intracellular compartment 40 during hemodialysis. Although the volume of water that transfers from the extracellular compartment 50 to the intracellular compartment 40 during hemodialysis may be taken into consideration, it is negligibly small, so that the post-hemodialysis extracellular fluid volume $_{ecf}Ve$ can be accurately calculated even though it is ignored.

While specific examples of the present disclosure have been described above in detail, these examples are merely illustrative and place no limitation on the scope of the patent claims. The technology described in the patent claims also encompasses various changes and modifications to the specific examples described above. The technical elements explained in the present description or drawings provide technical utility either independently or through various combinations. The present disclosure is not limited to the combinations described at the time the claims are filed.

In the above embodiment, the dialyzer overall mass transfer-area coefficient for uric acid is calculated by substituting the membrane area of the actually used dialyzer into the experiment formula indicating the relationship between the dialyzer's membrane area and the dialyzer overall mass transfer-area coefficient for uric acid. However, this is merely an example. A table in which each of various different dialyzer's membrane areas is associated with its corresponding overall mass transfer-area coefficient for uric acid may be created in advance, and an overall mass transfer-area coefficient for uric acid associated with the actually used dialyzer's membrane area may be read from the table.

**Claims**

1.  An extracellular fluid volume calculator (10) comprising:

    a processor (12); and
    a memory storing computer-readable instructions therein,
    wherein the computer-readable instructions, when executed by the processor (12), cause the extracellular fluid volume calculator (10) to execute:

    acquiring a membrane area of a dialyzer used for hemodialysis; and
    calculating a post-hemodialysis extracellular fluid volume based on a difference between a pre-hemodialysis amount of uric acid and a post-hemodialysis amount of uric acid, wherein a removal amount of uric acid removed by hemodialysis is calculated based on a dialyzer overall mass transfer-area coefficient for uric acid, and the dialyzer overall mass transfer-area coefficient for uric acid is calculated based on the membrane area of the dialyzer.

2.  The extracellular fluid volume calculator (10) according to claim 1, wherein
    the computer-readable instructions, when executed by the processor (12), cause the extracellular fluid volume calculator (10) to further execute: acquiring a plasma flow rate passing through the dialyzer and a dialysate flow rate passing through the dialyzer, and
    calculating the removal amount of uric acid based on a dialyzer clearance for uric acid, wherein the dialyzer clearance for uric acid is calculated based on the plasma flow rate and the dialysate flow rate acquired by the acquirement unit and the calculated dialyzer overall mass transfer-area coefficient for uric acid.

3.  A method for calculating an extracellular fluid volume, the method comprising:

    an acquirement step of acquiring a membrane area of a dialyzer used for hemodialysis; and
    a calculation step of calculating a post-hemodialysis extracellular fluid volume based on a difference between a pre-hemodialysis amount of uric acid and a post-hemodialysis amount of uric acid,
    wherein
    the calculation step comprises:

    a first calculation step (S16) of calculating a dialyzer overall mass transfer-area coefficient for uric acid based on the membrane area of the dialyzer acquired in the acquirement step; and
    a second calculation step (S20) of calculating a removal amount of uric acid removed by hemodialysis based on the dialyzer overall mass transfer-area coefficient for uric acid calculated in the first calculation step.

# FIG. 1

```
                                                    ⌐12
                                              ┌────────────────────────┐
                                              │                 ⌐14    │
                                              │  ┌──────────────────┐  │
                                              │  │ Patient's Information Storage Unit │  │
                                              │  └──────────────────┘  │
                                              │                 ⌐16    │
                                              │  ┌──────────────────┐  │
                                              │  │ Hemodialysis Information Storage Unit │  │
                                              │  └──────────────────┘  │
                                              │                 ⌐18    │
                                              │  ┌──────────────────┐  │
                                              │  │ Calculation Method Storage Unit │  │
                                              │  └──────────────────┘  │
                                              │                 ⌐20    │
                                              │  ┌──────────────────┐  │
                                              │  │   Calculation Unit   │  │
                                              │  └──────────────────┘  │
                                              └────────────────────────┘
```

Patient's Information Storage Unit

Hemodialysis Information Storage Unit

Calculation Method Storage Unit

Calculation Unit

Interface

# FIG. 2

40      50

Water ⟷ Water ⟷

Urea ⟷ Urea ⟷

Uric Acid ⇲⇱ Uric Acid ⟷

Sodium ⇲⇱ Sodium ⟷

42   52   56   54

FIG. 3

y=76.306ln(x)+184.23
$R^2$=0.97

# FIG. 4

# FIG. 5

```
                    ┌─────────────┐
                    │    Start     │
                    └──────┬──────┘
                           │                    S12
          ┌────────────────▼────────────────────┐
          │ Acquire Various Types of Information │
          │     About Hemodialysis Patient       │
          └────────────────┬────────────────────┘
                           │                    S14
          ┌────────────────▼────────────────────┐
          │ Acquire Various Types of Information │
          │        About Hemodialysis            │
          └────────────────┬────────────────────┘
                           │                    S16
          ┌────────────────▼────────────────────┐
          │     Calculate Dialyzer Overall Mass  │
          │ Transfer-Area Coefficient for Uric Acid │
          └────────────────┬────────────────────┘
                           │                    S18
      ┌───────────────────▼─────────────────────────┐
      │ Calculate Clearance for Uric Acid of Dialyzer │
      └───────────────────┬─────────────────────────┘
                           │                    S20
       ┌──────────────────▼──────────────────────┐
       │   Calculate Removal Amount of Uric Acid   │
       └──────────────────┬──────────────────────┘
                           │                    S22
          ┌────────────────▼────────────────────┐
          │     Calculate Post-Hemodialysis      │
          │     Extracellular Fluid Volume       │
          └────────────────┬────────────────────┘
                           │
                    ┌──────▼──────┐
                    │     End      │
                    └─────────────┘
```

## INTERNATIONAL SEARCH REPORT

International application No.

PCT/JP2019/032665

### A. CLASSIFICATION OF SUBJECT MATTER
Int.Cl. A61M1/16(2006.01)i

According to International Patent Classification (IPC) or to both national classification and IPC

### B. FIELDS SEARCHED
Minimum documentation searched (classification system followed by classification symbols)
Int.Cl. A61M1/14-1/18

| Documentation searched other than minimum documentation to the extent that such documents are included in the fields searched | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| P, A | WO 2019/138917 A1 (NIPRO CORP.) 18 July 2019, paragraphs [0047]-[0077] (Family: none) | 1-3 |
| A | IOLKO, Mariusz et al., "Accuracy of hemodialysis modeling", Kidney International, 2000, vol. 57, no. 3, pp. 1152-1163 | 1-3 |
| A | 金森敏幸，酒井清孝，＂短時間頻回透析の有用性に関する工学的検討＂，人工臓器, 1991, vol. 20, no. 5, pp. 14111420, (KANAMORI, T., SAKAI, K., "Therapeutic Usefulness of Short-time and Frequent Dialysis Based on Kinetic Modeling", Jinko Zoki) | 1-3 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 04 October 2019 (04.10.2019) | 21 October 2019 (21.10.2019) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2019/032665

C (Continuation). DOCUMENTS CONSIDERED TO BE RELEVANT

| Category* | Citation of document, with indication, where appropriate, of the relevant passages | Relevant to claim No. |
|---|---|---|
| A | JP 57-183855 A (ASAHI MEDICAL KK) 12 November 1982, page 2, lower left column, line 11 to page 3, upper right column, line 14, page 4, upper right column, line 16 to page 5, lower right column, line 2, fig. 1-4 (Family: none) | 1-3 |
| A | 峰島三千男, "2．ダイアライザ開発の方向性", 日本透析医学会雑誌, 2011, vol. 44, no. 6, pp. 536-537, non-official translation (MINESHIMA, Michio, "2. Directions of development of dialyzer", Journal of Japanese Society for Dialysis Therapy) | 1-3 |
| A | 山下明泰, 田中健一, 日台英雄, "血液透析膜の薄膜化と透析器形状", 日本透析療法学会雑誌, 1985, vol. 18, no. 5, pp. 519-523, (YAMASHITA, Akihiro, TANAKA, Kenichi, HIDAI, Hideo, "Thinner dialysis membrane and long type dialyzer module effecting solute and water removal performances", Journal of Japanese Society for Dialysis Therapy) | 1-3 |

Form PCT/ISA/210 (continuation of second sheet) (January 2015)

## INTERNATIONAL SEARCH REPORT

| International application No. |
| --- |
| PCT/JP2019/032665 |

<Regarding the subject of the search>

Claim 1 indicates that "said uric acid overall mass transfer-area coefficient is calculated on the basis of the membrane area of said dialyzer acquired from the acquisition part," and claim 3 indicates that "uric acid overall mass transfer-area coefficient is calculated on the basis of the membrane area of said dialyzer acquired in the acquisition step."

On this point, paragraph [0009] of the description indicates that "however, regarding all the dialyzer used in dialysis, performing such ex vivo experiments for the calculation of the uric acid overall mass transfer-area coefficient of the uric acid is not practical. The present inventors earnestly examined this, as a result, it is determined that correlation between the uric acid overall mass transfer-area coefficient and the membrane area of the dialyzer is present. Therefore, the uric acid overall mass transfer-area coefficient can be calculated from the membrane area of the dialyzer," more specifically, paragraphs [0037]-[0039] indicate that a correlation represented by [equation 13] $y=76.306\ln(x)+184.23$ between the membrane area (x) of the dialyzer and the uric acid overall mass transfer-area coefficient (y) as shown in [table 1] is recognized, and the uric acid overall mass transfer-area coefficient can be calculated by entering the membrane area of the dialyzer used in dialysis in equation 13.

However, regarding four combinations of the membrane area (x) of the dialyzer and the uric acid overall mass transfer-area coefficient (y) as shown in [table 1], the roughly applied approximate curve by equation 13 doesn't mean that the correlation represented by equation 13 between the membrane area (x) of the dialyzer and the uric acid overall mass transfer-area coefficient (y) is recognized.

Furthermore, the overall mass transfer-area coefficient KoA of the dialyzer is a multiplication of the overall mass transfer coefficient Ko which represents substance permeability through a dialysis membrane and the effective membrane area A of the dialyzer (if necessary, see document: MINESHIMA, Michio, "2. Directions of development of dialyzer", Journal of Japanese Society for Dialysis Therapy, 2011, vol. 44, no. 6, pp. 536-537), and even when having the same membrane area, if the dialyzer has a different overall mass transfer coefficient Ko or effective membrane area A, KoA can take a different value, and thus even though a correlation represented by equation 13 between the membrane area of the dialyzer and the uric acid overall mass transfer-area coefficient is recognized regarding a specific dialyzer, it cannot consider that the equation represents a correlation between the membrane area of another dialyzer and the uric acid overall mass transfer-area coefficient. It should be considered that this is supported from the description in that even though the membrane of the dialyzer has the same material and almost the same area, when the specification and module shape of the membrane are largely different, dependence characteristics on a dialyzate flow rate of clearance is also largely different, and solute-removing effect which can be obtained is different even using in the same condition of treatment, as a result of the specific experiment, with regard to uric acid clearance and uric acid removal rate, the score of a new model dialyzer which employs a thin membrane and an elongated module and has a membrane area of 1.5 $m^2$ is higher than the score of an old model dialyzer which has a membrane area of 1.6 $m^2$ in document: YAMASHITA, Akihiro, TANAKA, Kenichi, HIDAI, Hideo, "Thinner dialysis membrane and long type dialyzer module effecting solute and water removal performances", Journal of Japanese Society for Dialysis Therapy, 1985, vol. 18, no. 5, pp. 519-523.

Form PCT/ISA/210 (extra sheet) (January 2015)

**INTERNATIONAL SEARCH REPORT**

International application No.

PCT/JP2019/032665

Accordingly, it can be said that, regardless of dialyzer of paragraph [0033] which is the basis of equation 13, with regard to the dialyzer different from this, whether equation 13 is appropriate to the correlation between the membrane area and the uric acid overall mass transfer-area coefficient is not apparent. (in addition, paragraph [0050] indicates that it is identified that with regard to a few dialyzer, uric acid removal amount can be accurately calculated on the basis of the uric acid overall mass transfer-area coefficient calculated from the membrane area using equation 13, but it is not identified whether the uric acid overall mass transfer-area coefficient calculated from the membrane area using equation 13 corresponds to the actually measured uric acid overall mass transfer-area coefficient. Since it is common technical knowledge that the measured value of uric acid removal amount is largely affected by measurement errors, even though there is no distinct difference between the calculated uric acid removal amount and the measured uric acid removal amount, it cannot be always considered that the uric acid overall mass transfer-area coefficient calculated from the membrane area using equation 13 corresponds to the actual uric acid overall mass transfer-area coefficient.)

Therefore, for calculating the uric acid overall mass transfer-area coefficient from the membrane area using equation 13 in the invention in claims 1-3, validity with regard to a dialyzer different from the dialyzer of paragraph [0033] is not secured, and thus it should be said that the invention is not fully supported by the description under PCT Article 6.

Moreover, since the invention in claims 1-3 is not limited to using equation 13, the description does not disclose and suggest that the correlation between the membrane area of a dialyzer and the uric acid overall mass transfer-area coefficient is represented by an equation other than equation 13. In addition, in light of common technical knowledge, it is not recognized that there is a correlation between the membrane area of a dialyzer and the uric acid overall mass transfer-area coefficient, wherein the uric acid overall mass transfer-area coefficient is uniquely determined by only the membrane area of the dialyzer, and thus even if, regarding a specific dialyzer, a correlation represented by an equation other than equation 13 between the membrane area of the dialyzer and the uric acid overall mass transfer-area coefficient can be found, it is apparent that the equation cannot represent a correlation between the membrane area of another dialyzer and the uric acid overall mass transfer-area coefficient.

Therefore, for using an equation other than equation 13 in the invention in claims 1-3, the invention is not supported by the description under PCT Article 6 at all.

Moreover, in the practice of the invention in claims 1-3, there is a need to identify whether the equation (e.g., equation 13) for calculating the uric acid overall mass transfer-area coefficient on the basis of the membrane area of the dialyzer is appropriate to any dialyzer, and this makes those skilled in the art perform excessive experiments. Thus, the description lacks clear and sufficient disclosure stipulated in PCT Article 5 regarding the invention in claims 1-3.

As above, since a meaningful search could not be carried out for calculating the uric acid overall mass transfer-area coefficient from the membrane area of the dialyzer using an equation other than equation 13, the search was carried out for the invention in claims 1-3, limiting to calculating the uric acid overall mass transfer-area coefficient from the membrane area of the dialyzer using the equation represented by equation 13.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUIK A et al.** Blood pressure control and fluid state in patients on long treatment time dialysis. *J Am Soc Nephrol,* 1994, vol. 5, 521
- **JAEGER JQ ; MEHTA RL.** Assessment of Dry Weight in Hemodialysis: An Overview. *J Am Soc Nephrol,* 1999, vol. 10, 392-403
- **CHARRA B et al.** Clinical assessment of dry weight. *Nephrol Dial Transplant,* 1996, vol. 11 (2), 16-19
- **GUNAL AI.** How to determine 'dry weight'?. *Kidney Int,* 2013, vol. 3, 377-379
- **ERIKO ISHII et al.** The target range of plasma ANP level for dry weight adjustment in HD patients. *Journal of Japanese Society for Dialysis Therapy,* 2004, vol. 37, 1417-1422
- **BRANDT RR et al.** Atrial natriuretic peptide in heart failure. *J Am Coll Cardiol,* 1993, vol. 22 (A), 86A-92A
- **NAGENDRA S et al.** A comparative study of plasma uric acid, erythrocyte uric acid and urine uric acid levels in type 2 diabetic subjects. *Merit Research Journal,* 2015, vol. 3, 571-574
- **ERIC DESCOMBES et al.** Diffusion kinetics of urea, creatinine and uric acid in blood during hemodialysis. *Clinical implications. Clinical Nephrology,* 1993, vol. 40, 286-295